# EUROPÄISCHE PATENTSCHRIFT

(11) **EP 2 139 537 B1**
(45) Veröffentlichungstag und Bekanntmachung des Hinweises auf die Patenterteilung: **15.09.2010**
(21) Anmeldenummer: 08735187.0
(22) Anmeldetag: 11.04.2008
(51) Int. Cl.: A61M 1/28

(54) **Verfahren zur Vorbereitung einer Behandlungsmaschine und Behandlungsmaschine**
Method for preparing a treatment machine and treatment machine
Procédé de préparation d'une machine de traitement et machine de traitement

(30) Priorität: 18.04.2007 DE 102007018362
(43) Veröffentlichungstag der Anmeldung: 06.01.2010
(73) Patentinhaber: Fresenius Medical Care Deutschland GmbH, 61352 Bad Homburg (DE)
(72) Erfinder: WICH-HEITER, Joachim, 97273 Kürnach (DE)
(74) Vertreter: Laufhütte, Dieter
(86) Internationale Anmeldenummer: PCT/EP2008/002896
(87) Internationale Veröffentlichungsnummer: WO 2008/128664

(56) Entgegenhaltungen:
- EP-A- 0 815 882
- WO-A-2005/042065
- US-A- 4 718 022

## Beschreibung

Die vorliegende Erfindung betrifft ein Verfahren zur Vorbereitung einer Behandlungsmaschine zur Behandlung einer medizinischen Flüssigkeit, welche eine Ankoppelfläche mit Aktoren umfaßt, wobei eine Kassette aus einem Hartteil mit flüssigkeitsführenden Kanälen, welche von einer flexiblen Folie abgedeckt sind, an die Ankoppelfläche der Behandlungsmaschine ankoppelbar ist, so dass die Aktoren durch Herabdrücken der flexiblen Folie in die Kassette den Flüssigkeitsfluss in den flüssigkeitsführenden Kanälen steuern können. Die vorliegende Erfindung umfaßt weiterhin eine entsprechende Behandlungsmaschine mit einer Steuerung zur Durchführung des erfindungsgemäßen Verfahrens. Insbesondere handelt es sich bei der erfindungsgemäßen Behandlungsmaschine um eine Behandlungsmaschine zur Dialyse, insbesondere zur Peritonealdialyse.

Eine bekannte Behandlungsmaschine zur Dialyse ist in WO2005/042065 beschrieben.

Beim Aufrüsten einer solchen Behandlungsmaschine muss die benötigte Kassette, welche als Einwegartikel ausgeführt ist, in die Maschine eingelegt und an die Koppelfläche gekoppelt werden. Die Disposable-Kassette weist hierzu eine Ankoppelebene zur Ankopplung an die Ankoppelfläche der Behandlungsmaschine auf, welche flächig mit einer dünnen flexiblen Polymerfolie verschlossen ist, so dass verschiedene Aktoren und Sensoren der Behandlungsmaschine an die äußere Folienoberfläche ankoppeln können. Bei den Aktoren kann es sich dabei um Ventilstößel handeln. Die Ventilstößel drücken an verschiedenen Stellen die flexible Polymerfolie in entsprechende flüssigkeitsführende Kanäle oder Vertiefungen der Kassette und dichten so die flüssigkeitsführenden Kanäle ab.

Aus werkstofftechnischen und hygienischen Gründen war es bisher nicht möglich, eine solche Behandlungsmaschine, und zwar insbesondere eine Maschine für die automatisierte Peritonealdialyse (APD), mit eingelegter Kassette nach dem Aufrüsten in einem Wartemodus stehen zu lassen. Die federbelasteten Ventilstößel können bei dauerhafter Druckeinwirkung des Stößels auf die Disposablefolie eine visko-elastische Verformung (Kriechverformung) der Folie verursachen, die so genannte Sackbildung. Eine solche Sackbildung muß aber verhindert werden, weil ansonsten die Funktionsfähigkeit der Disposable-Kassette bzw. der durch die Aktoren im Zusammenspiel mit der Folie gebildeten Ventile beeinträchtigt werden kann. Peritonealdialysepatienten, die mittels Heimperitonealdialyse behandelt werden, müssen daher nach den aus dem Stand der Technik bekannten Verfahren Einschränkungen ihrer Bewegungsfreiheit hinnehmen, weil sie gezwungen sind, unmittelbar nach der Vorbereitung (Aufrüstung) der Dialysemaschine sofort die mehrere Stunden andauernde Behandlung zu beginnen. Dies wird von den Patienten und der Fachwelt allgemein als gegeben hingenommen und nicht weiter hinterfragt.

Zwar ist es bekannt, dass ein erster Zyklus aus Auslauf und Einlauf während des Tages durchgeführt und die automatisierte Peritonealdialysebehandlung daraufhin unterbrochen werden kann. Auch in einem solchen Fall tritt die oben beschriebene Problematik des Werkstoffkriechens der Folie auf. Als Gegenmaßnahme gegen die Kriechverformung der Disposablefolie wird deshalb während des Unterbrechungsmodus entsprechend einer bestimmten Logik automatisch eine regelmäßige Bewegung der Ventilstößel ausgeführt. Durch diese Bewegung wird verhindert, dass sich die Folie an den Ventilstellen durch eine dauerhafte Druckeinwirkung des Stößels verformt. Der zusätzliche Zyklus am Tage verbessert zwar die Wirksamkeit der Peritonealdialyse, schränkt den Patienten in seiner Tagesgestaltung aber weiter ein. Insbesondere muß bei bekannten Behandlungsgeräten immer direkt nach dem Aufrüsten der Behandlungsmaschine mit der Behandlung begonnen werden. Das Aufrüsten des Dialysegerätes ist aber relativ zeitraubend und aufwendig und besteht aus den Schritten Systemcheck, Einlegen der Kassette, Einlegen der Lösungsbeutelkonnektoren, Anschluß der Drainageleitung, Füllen der Kassette und der Schläuche. Deshalb wäre es hier zur Verbesserung der Lebensqualität der Patienten wünschenswert, die Abläufe flexibler zu gestalten und die Einschränkung der Bewegungsfreiheit des Patienten auf das unbedingt notwendige Maß zu reduzieren, so dass die Patienten flexibler am gesellschaftlichen Leben teilnehmen können.

Aufgabe der vorliegenden Erfindung ist es daher, ein Verfahren zur Vorbereitung einer Behandlungsmaschine sowie eine entsprechende Behandlungsmaschine zur Verfügung zu stellen, welche eine flexiblere Gestaltung der Abläufe bei der Vorbereitung der Behandlungsmaschine erlauben.

Erfindungsgemäß wird diese Aufgabe von einem Verfahren zur Vorbereitung einer Behandlungsmaschine gemäß Anspruch 1 gelöst. dabei wird nach dem Aufrüsten der Behandlungsmaschine und vor dem Behandlungsbeginn in einen stand-by Modus geschaltet, bei welchem die Aktoren abwechselnd bewegt werden, um die flexible Folie zu entlasten. Durch den erfindungsgemäßen stand-by Modus vor Behandlungsbeginn ist es erstmals möglich, die zeitaufwendige Aufrüstung der Behandlungsmaschine zeitlich völlig von der Behandlung selbst zu entkoppeln. Im Gegensatz zu Behandlungsmaschinen gemäß dem Stand der Technik, bei welchen eine Unterbrechung zwischen dem Aufrüsten und der Behandlung sogar ausdrücklich verboten war und diese Einschränkung der Bewegungsfreiheit des Patienten als gegeben hingenommen wurde, ist nun eine deutliche Flexibilisierung des Ablaufs möglich. Durch das Bewegen der Ventilstößel ist es dabei möglich, den stand-by Modus auch über sehr lange Zeiträume beizubehalten, ohne dass durch eine dauerhafte Druckeinwirkung der Aktoren eine Kriechverformung der Folie zu befürchten wäre.

Während es gemäß dem Stand der Technik nicht nur als unumgänglich, sondern sogar als sinnvoll galt, im Anschluß an die Aufrüstung sofort mit der Behandlung zu beginnen, um die Gesamtdauer gerade nicht durch zusätzliche Wartezeiten zu verlängern, können Aufrüstung und Behandlung durch den erfindungsgemäßen stand-by Modus erstmals komplett voneinander entkoppelt werden, was dem Patienten eine erhebliche Erleichterung in der Gestaltung seines Tagesablaufes ermöglicht. Insbesondere sind es die mit dem erfindungsgemäßen Verfahren möglichen sehr langen stand-by Zeiten, welche einen stand-by Modus vor Behandlungsbeginn überhaupt erst sinnvoll machen. Eine Anforderung nach längeren stand-by Zeiten zwischen Aufrüstung und Behandlungsbeginn bestand dagegen nach dem Stand der Technik erst gar nicht, da der Patient die Behandlung ja so schnell wie möglich absolvieren wollte. Vorteilhafterweise wird dagegen in der vorliegenden Erfindung der stand-by Modus 10 Stunden, weiterhin vorteilhafterweise 15 Stunden beibehalten.

Der Patient kann mit Hilfe der vorliegenden Erfindung beispielsweise irgendwann im Laufe des Tages die Behandlungsmaschine aufrüsten und in den stand-by Modus versetzen, z. B. abends am gesellschaftlichen Leben teilnehmen und bei Beginn der Nachtbehandlung ohne weitere zeitaufwendige Vorbereitungen sofort konnektieren und mit der Nachtbehandlung beginnen. Die Behandlung wird dann in bekannter Weise während des Schlafes automatisch durchgeführt.

Vorteilhafterweise werden dabei im stand-by Modus die Aktoren so bewegt, dass keine vorher verschlossenen Flußwege geöffnet werden. Die Fluidverbindungen innerhalb der Kassette werden so während des stand-by Modus im wesentlichen nicht verändert, die Kriechverformung der Folie durch eine abwechselnde Verwendung z. B. benachbarter Ventilstößel jedoch effektiv verhindert.

Weiterhin vorteilhafterweise werden die Aktoren dabei in regelmäßigen Abständen, weiterhin vorteilhafterweise in Abständen von 5 bis 20 Minuten bewegt. Hierdurch ist eine sichere Verhinderung einer Kriechverformung gewährleistet, wobei dieser stand-by Modus auch über lange Warteperioden aufrecht erhalten werden kann, ohne die Folie zu beschädigen.

Die vorliegende Erfindung umfaßt weiterhin ein Verfahren zur Vorbereitung einer Behandlungsmaschine zur Behandlung einer medizinischen Flüssigkeit, wobei nach dem Aufrüsten der Behandlungsmaschine und vor dem Behandlungsbeginn in einen stand-by Modus geschaltet wird, bei welchem ein Überdruck im System hergestellt wird. Dadurch, dass erfindungsgemäß während des stand-by Modus ein Überdruck in der Kassette hergestellt wird, wird eine Verkeimung über die angeschlossenen Konnektoren wirksam verhindert. Dabei ist offensichtlich, dass eine solche Verhinderung der Verkeimung unabhängig von dem erfindungsgemäßen Bewegen der Aktoren von großem Vorteil ist. In besonderer Weise vorteilhaft ist aber gerade eine Kombination beider Verfahren, da hierdurch auch sehr lange stand-by Zeiten sicher ermöglicht werden.

Die vorliegende Erfindung umfaßt weiterhin Behandlungsmaschinen, deren Steuerung zur Durchführung der erfindungsgemäßen Verfahren ausgelegt ist. Die vorliegende Erfindung umfaßt daher gemäß Anspruch 6 weiterhin eine Behandlungsmaschine zur Behandlung einer medizinischen Flüssigkeit, mit einer Ankoppelfläche mit Aktoren und mit einer Steuerung, wobei eine Kassette aus einem Hartteil mit flüssigkeitsführenden Kanälen, welche von einer flexiblen Folie abgedeckt sind, an die Ankoppelfläche der Behandlungsmaschine ankoppelbar ist, so dass die Aktoren durch Herabdrükken der flexiblen Folie in die Kassette den Flüssigkeitsfluss in den flüssigkeitsführenden Kanälen steuern können. Erfindungsgemäß weist die Steuerung der Behandlungsmaschine dabei einen nach dem Aufrüsten der Behandlungsmaschine und vor dem Behandlungsbeginn einschaltbaren stand-by Modus auf, bei welchem die Aktoren abwechselnd bewegt werden, um die flexible Folie zu entlasten. Hierdurch sind die bereits mit Bezug auf das Verfahren beschriebenen Vorteile gegeben. Insbesondere ist es so möglich, vor der erstmaligen Patientenkonnektion die aufgerüstete Dialysemaschine in einen Zustand stellen zu können, der es erlaubt, erst Stunden später den Patienten zu konnektieren und dann direkt die Behandlung starten zu können. Während dieses stand-by Modus vor dem Behandlungsbeginn werden dabei die bereits oben beschriebenen Gegenmaßnahmen bezüglich der Sackbildung in der Folie durchgeführt.

Insbesondere werden dabei im stand-by Modus vorteilhafterweise die Aktoren so bewegt, dass keine vorher verschlossenen Flusswege geöffnet werden. Weiterhin vorteilhafterweise werden im stand-by Modus die Aktoren in regelmäßigen Abständen, vorteilhafterweise in Abständen von 5 bis 20 Minuten bewegt.

Weiterhin vorteilhafterweise umfaßt die vorliegende Erfindung auch eine Behandlungsmaschine, bei welcher während dem stand-by Modus Gegenmaßnahmen gegen eine Verkeimung der Kassette durchgeführt werden. Insbesondere umfaßt die vorliegende Erfindung hierzu eine Behandlungsmaschine, bei welcher die Steuerung einen nach dem Aufrüsten der Behandlungsmaschine und vor dem Behandlungsbeginn einschaltbaren stand-by Modus aufweist, in welchem die Steuerung einen Überdruck im System herstellt. Hierdurch wird wiederum wie beim erfindungsgemäßen Verfahren eine Verkeimung des Sets wirksam verhindert. Wiederum kann eine solche Behandlungsmaschine unabhängig von der Bewegung der Aktoren während des stand-by Modus von großem Vorteil sein, wobei jedoch gerade eine Kombination beider Steuerungsmerkmale von besonderem Vorteil ist.

Weiterhin vorteilhafterweise schaltet die Steuerung der erfindungsgemäßen Behandlungsmaschine nach dem Aufrüsten automatisch in den stand-by Modus. Insbesondere schaltet die Steuerung automatisch in den stand-by Modus, wenn nicht innerhalb einer vorgegebenen Zeit mit der Behandlung begonnen wird. Hierdurch wird sichergestellt, dass die Gegenmaßnahmen bezüglich Sackbildung und Verkeimung durchgeführt werden, wenn nicht mit der Behandlung begonnen wird. Das automatische Schalten in den stand-by Modus gewährleistet dabei, dass Bedienfehler durch den Patienten erst gar nicht auftreten können.

Weiterhin vorteilhafterweise ist die erfindungsgemäße Behandlungsmaschine eine Behandlungsmaschine zur Peritonealdialyse, insbesondere eine Behandlungsmaschine zur automatischen Peritonealdialyse.

Die vorliegende Erfindung wird nun anhand eines Ausführungsbeispiels sowie anhand von Zeichnungen näher dargestellt.

Dabei zeigen
- Figur 1: eine Disposable-Kassette,
- Figuren 2a bis 2c: die Sackbildung beim Verschließen der flüssigkeitsführenden Kanäle der Kassette durch dauerhaftes Einwirken der Akto- ren,
- Figuren 3a und 3b: unterschiedliche Ventilstößeistellungen während des stand-by Modus gemäß der vorliegenden Erfindung und
- Fig. 4: eine Standardbehandlung bei der Peritonealdialyse.

In der vorliegenden Erfindung werden sowohl das Aufrüsten der Behandlungsmaschine als auch die Behandlung des Patienten wie mit Behandlungsmaschinen nach dem Stand der Technik durchgeführt, wobei es nun jedoch durch den erfindungsgemäßen stand-by Modus erstmals möglich ist, beide Vorgänge zeitlich zu entkoppeln.

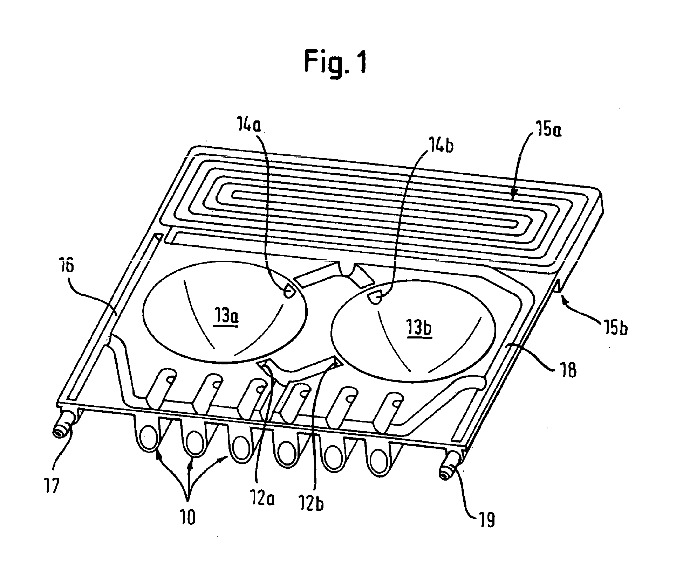

In Figur 1 ist nun zunächst die sowohl bei bekannten Behandlungsmaschinen als auch bei der vorliegenden Erfindung zum Einsatz kommende Kassette aus einem Hartteil mit flüssigkeitsführenden Kanälen gezeigt, welche von einer nicht dargestellten flexiblen Folie abgedeckt sind. Die Kassette weist dabei links bzw. rechts an der Vorderseite einen Anschluß 17 für den Patientenschlauch sowie einen Anschluß 19 für den Drainageschlauch auf. Dazwischen befinden sich Setstutzen 10 zum Anschluß von Lösungsbeuteln. Die Kassette weist Kanäle 18 für das Dialysat vom Patienten sowie Kanäle 16 für die Dialysierflüssigkeit zum Patienten auf.

Die Flüssigkeit wird dabei über eine linke Pumpenkammer 13a und eine rechte Pumpenkammer 13b, welche als halbkugelartige Vertiefungen im Hartteil ausgeführt sind, gepumpt. Die Pumpenkammern 13a und 13b weisen jeweils Einlässe 12a und 12b sowie Auslässe 14a und 14b auf. Des weiteren ist in der Kassette ein oberer Durchlaufheizungsbereich 15a und ein unterer Durchlaufheizungsbereich 15b vorgesehen, in welchem die durchfließende Dialysierflüssigkeit mit entsprechenden Heizelementen der Behandlungsmaschine in Kontakt steht und auf Körpertemperatur aufgewärmt wird. Das Hartteil der Kassette mit den flüssigkeitsführenden Kanälen und den halbkugelförmigen Pumpenkammern 13a und 13b ist von einer transparenten und wärmeleitfähigen flexiblen Folie überzogen.

Durch das wechselseitige Drücken der Pumpenkolben auf der Ankoppelfläche der Behandlungsmaschine, durch welches die Folie in die Pumpenkammern 13a und 13b gedrückt wird, wird ein kontinuierliches Pumpen realisiert. Die Kanalwege werden dabei von Ventilen gesteuert, deren Ventilstößel die flexible Folie in die entsprechenden Flusskanäle und Vertiefungen und Kassette hineindrücken und so die Flusswege in der Kassette verschließen. Hierdurch können verschiedene Flusswege für die Dialyseflüssigkeit realisiert werden, wobei die Dialyseflüssigkeit z.B. aus den angeschlossenen Lösungsbeuteln in die Patientenleitung gepumpt werden kann.

Nun werden die zum Aufrüsten der Behandlungsmaschine notwendigen Schritte beschrieben, mit welchen der Patient die Behandlungsmaschine nach dem Einschalten vorbereiten muß, wobei er durch eine Bildschirmanzeige geführt wird. Im ersten Schritt wird ein steriles Set mit einer Disposable-Kassette in das Gerät eingelegt. Dann werden Lösungsbeutel mit der Dialyseflüssigkeit an die Kassette angeschlossen. Hierzu werden die Konnektoren der Lösungsbeutel in eine Schublade eingelegt und später von einer Konnektorleiste der Schublade auf die zylindrischen Setstutzen 10 der Kassette geschoben. Hierdurch werden die Setstutzen von den Konnektoren durchstochen und eine fluidische Verbindung zwischen Lösungsbeutel und Kassette hergestellt. Nun muß die Drainageleitung an das Set angeschlossen werden, sowie das Set und die Schläuche befüllt werden. Erst nun ist das Dialysegerät aufgerüstet, so dass der Patient durch Abschrauben einer Schutzkappe vom Patientenkonnektor und Anschrauben einer Katheterverlängerung konnektiert werden kann. Die flüssigkeiteführenden Kanäle in der Kassette werden nun über Ventilstößel verschlossen, um einen ungewollten Durchfluß durch die Kassette zu verhindern.

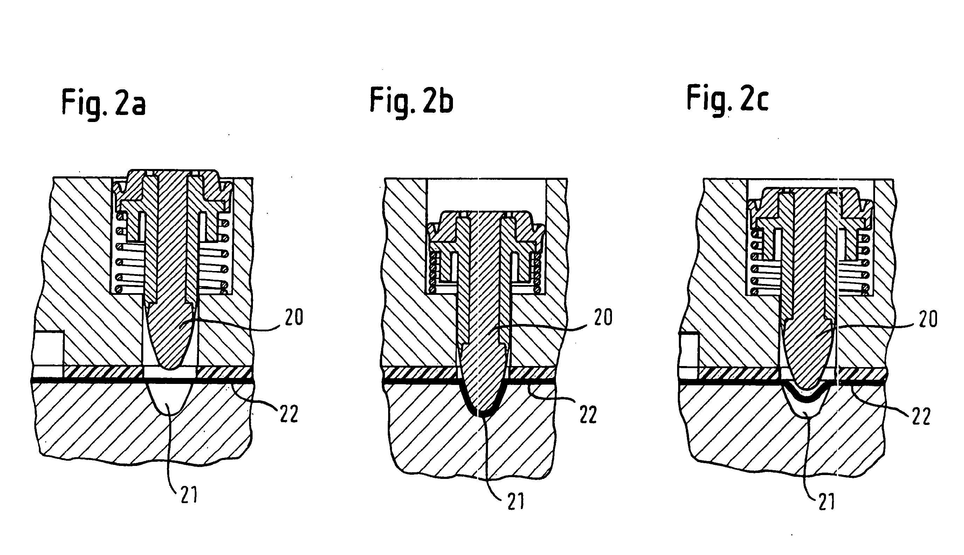

Die Ventilstößel dürfen dabei nicht über längere Zeit ununterbrochen auf die Folie einwirken, da sonst eine unerwünschte Kriechverformung der Folie einsetzt. In Figuren 2a bis 2c wird dabei gezeigt, wie ein maschinenseitiger Aktor 20 in Form eines Ventilstößels die Folie 22 in den flüssigkeitsführenden Kanal 21 hineindrückt und eine solche Kriechverformung bewirkt.

In Figur 2a befindet sich das Ventil im total geöffneten Zustand, in welchem der Ventilstößel 20 nicht mit der Folie 22 in Kontakt steht. Diese wird neben dem flüssigkeitsführenden Kanal 21 mit Dichtbereichen des Hartteils verpresst und verläuft oberhalb des flüssigkeitsführenden Kanals 21 eben. In Figur 2b ist der Ventilstößel 20 nun komplett in den flüssigkeitsführenden Kanal 21 hineingedrückt, so dass die Folie 22 maximal verformt ist und der flüssigkeitsführenden Kanal 21 verschlossen ist. Wird der Ventilstößel 20 nach längerer Belastungsdauer wieder entlastet, bleibt, wie in Figur 2c gezeigt ist, die Verformung der Folie bis zu einem gewissen Grad erhalten. Da die verformte Folie 22 an dieser Stelle einer Sackform ähnelt, spricht man hier von Sackbildung. Je länger der Stößel 20 in die Folie 22 drückt, desto bleibender kann die Verformung bzw. die Sackbildung sein.

Der durch den Fluß im flüssigkeitsführenden Kanal 21 entstehende Sog bzw. Druck kann den Foliensack bei geöffnetem Ventil dann so in den Kanal zwingen, dass es zu einer Verengung oder gar zu einem Verschluß kommen kann. Eine zu lange Verformung der Folie 22 durch den Ventilstößel 20 mit einhergehender Sackbildung kann so die Funktionsfähigkeit der Kassette beeinträchtigen oder ein ordnungsgemäßes Funktionieren sogar komplett ausschließen. Bei Geräten gemäß dem Stand der Technik mußte daher nach Abschluß des Füllvorganges sofort mit der Behandlung begonnen werden.

Damit das aufgerüstete Gerät auch für längere Zeit im aufgerüsteten Zustand verbleiben kann, so dass die Behandlung nicht sofort nach dem Aufrüsten begonnen werden muß, ist der erfindungsgemäße Stand-by Modus vorgesehen, während welchem die Ventilstößel regelmäßig bewegt werden, um die flexible Folie zu entlasten und so einer Sackbildung entgegen zu wirken. Durch diese Reduktion der dauerhaften Einwirkzeit des Ventilstößels auf die Folie kann verhindert werden, dass es zu einer bleibenden Verformung der Setfolie kommt.

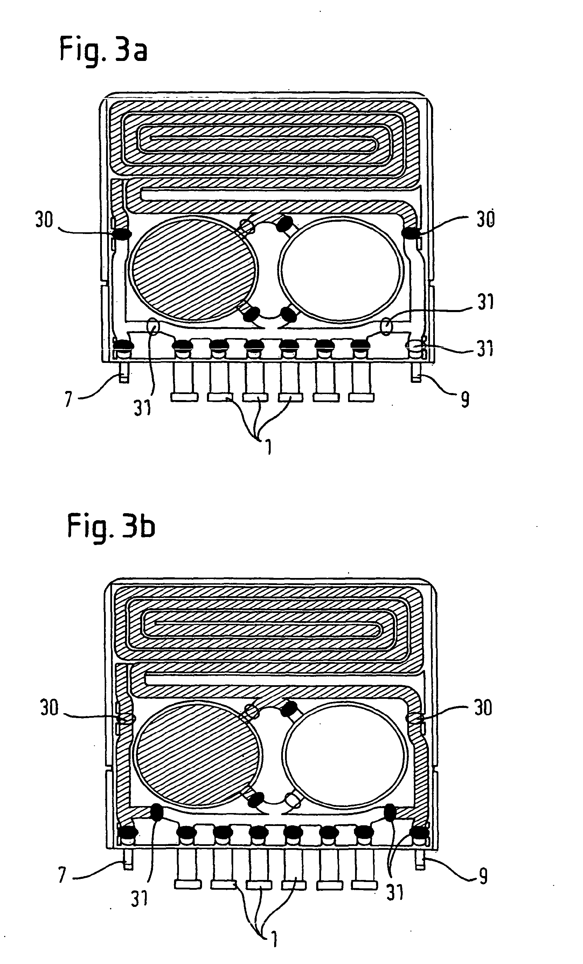

Wie anhand der Figuren 3a und 3b dargestellt, können dabei durch Benutzung von Ventilstößeln, welche nicht zur Steuerung des aktiven Flussweges nötig sind, unterschiedliche Ventilstößelstellungen verwendet werden, ohne dass sich der effektive Flussweg zwischen den äußeren Anschlüssen ändert. Hierbei können die Ventilstößel nach einem festgesetzten Zeitintervall, z. B. 10 Minuten, von der Setfolie entfernt und durch andere Ventilstößel ersetzt werden.

Figuren 3a und 3b zeigen dabei beispielhaft zwei unterschiedliche Ventilstößelstellungen, wobei die fluidisch miteinander in Verbindung stehenden Bereiche der Kassette jeweils schraffiert bzw. weiß dargestellt sind. In Figur 3a sind dabei die Ventile 30 geschlossen, während die Ventile 31 geöffnet sind. In Figur 3a sind daher die Ventilstößel der Ventile 30 in die Folie herabgedrückt, während die Ventilstößel der Ventile 31 hochgezogen sind und nicht mit der Folie in Kontakt stehen.

In Figur 3b sind dagegen die Ventile 30 geöffnet, während die Ventile 31 geschlossen sind. Bezüglich der Flusswege zwischen den äußeren Anschlüssen 7 für den Patienten, 9 für die Drainage und 10 für die Lösungsbeutel ergibt sich dagegen kein Unterschied. Durch ein abwechselndes Entlasten der Folie durch Öffnen der Ventile wird so sicher gestellt, dass keine dauerhafte Kriechverformung der Folie stattfindet.

Dadurch, dass während des erfindungsgemäßen stand-by Modus eine durchgehende Einwirkung der Ventilstößel auf die Folie durch das abwechselnde Bewegen der einzelnen Ventilstößel vermieden wird, ist auch eine lange Wartezeit von bis zu 15 Stunden zwischen Aufrüsten und Beginn der Behandlung bei dem erfindungsgemäßen Verfahren möglich. Zudem wird erfindungsgemäß weiterhin während des stand-by Modus ein geringer Überdruck in der Kassette hergestellt, um eine Verkeimung zu vermeiden.

Der Patient kann mit Hilfe der vorliegenden Erfindung beispielsweise irgendwann im Laufe des Tages die Behandlungsmaschine aufrüsten und in den stand-by Modus versetzen. Hierzu ist eine entsprechende Benutzerführung der Behandlungsmaschine vorgesehen, welche dem Patienten nach dem Aufrüsten der Behandlungsmaschine die Wahl zwischen einem sofortigen Beginn der Behandlung und dem erfindungsgemäßen stand-By Modus bietet. Dabei können auch weitere Dateneingaben wie z.B. die gewünschte stand-by Dauer (in Stunden) oder das gewünschte stand-by Ende (Uhrzeit) ermöglicht werden. Die Benutzerführung erfolgt dabei vorteilhafterweise über einen Touch-Screen. Vorteilhafterweise ist die Steuerung der Behandlungsmaschine weiterhin so ausgelegt, dass diese nach dem Aufrüsten automatisch in den stand-by Modus schaltet, wenn nicht nach einer gewissen Zeit mit der Behandlung begonnen wurde.

Der Patient kann durch den erfindungsgemäßen stand-By Modus nach dem Aufrüsten z. B. abends am gesellschaftlichen Leben teilnehmen und bei Beginn der Nachtbehandlung ohne weitere zeitaufwendige Vorbereitung sofort Konnektieren und mit der Nachtbehandlung beginnen. Die Behandlung kann dann bekannterweise wie weiter oben beschrieben während des Schlafes automatisch durchgeführt werden.

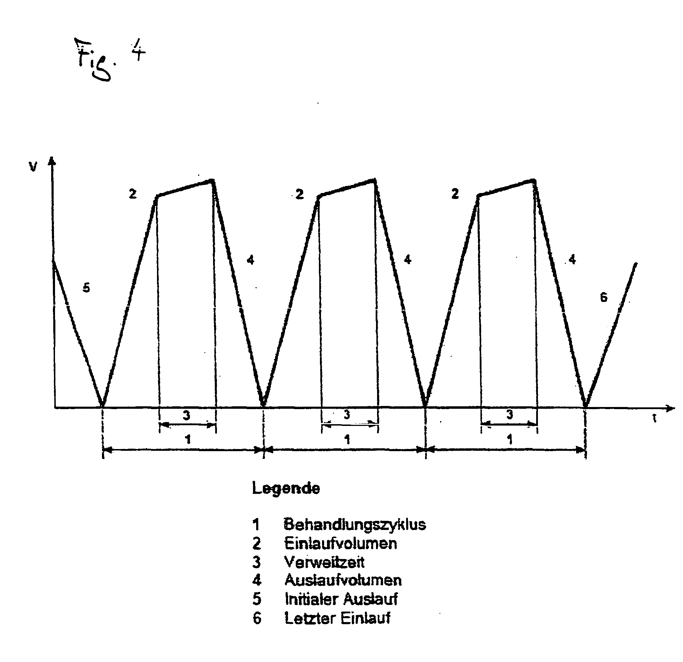

Eine Standardbehandlung, wie sie auch bei einer erfindungsgemäßen Behandlungsmaschine nach dem stand-by Modus z.B. während der Nacht durchgeführt wird, wird nun anhand von Figur 4 beschrieben. Da das Gerät bereits aufgerüstet ist, kann der Patient sofort mit der Dialysemaschine konnektiert werden. Hierzu entfemt er die Schutzkappe vom Patientenkonnektor und schraubt die Katheterverlängerung an.

Wie in Fig. 4 gezeigt, wird nun in einem Initialenauslauf 5 Dialyseflüssigkeit aus dem Bauchraum des Patienten herausgepumpt. Danach wird frische Flüssigkeit durch einen Einlauf 2 in den Bauchraum gepumpt, die für eine bestimmte Verweilzeit 3 dort verbleibt. Danach wird der Patient wieder mit einem Auslauf 4 geleert. Die Abfolge von Einlauf, Verweilzeit und Auslauf wird als Behandlungszyklus bezeichnet, welcher je nach Verschreibung wiederholt werden kann. Die Behandlung kann mit einem letzten Einlauf 6 beendet werden. Im Diagramm in Figur 4 ist dabei das Volumen V über die Zeit aufgetragen. Der initiale Auslauf 5 und der letzte Einlauf 6 können dabei optional von medizinischen Personal aktiviert werden. Nach dem Ende der Standardbehandiung behält der Patient dann seine Dialyseflüssigkeit bis zur nächsten Behandlung.

Neben der in Figur 4 dargestellten Standardbehandlung ist es zudem möglich, in einem alternativen Verfahren tagsüber die verbrauchte Dialyseflüssigkeit einmal gegen frische auszutauschen. Auch hierzu wird nach dem Anschließen der Patientenleitung die Dialyseflüssigkeit im Bauchraum des Patienten mit einem initialen Auslauf 5 herausgepumpt und durch frische Flüssigkeit, welche mit einem ersten Einlauf 2 in den Bauchraum gepumpt wird, ersetzt. Nun kann das Behandlungsverfahren für einen gewissen Zeitraum unterbrochen werden, während dem sich der Patient diskonnektiert und die Patientenleitung sowie die Katheterleitung verschließt. Der Patient kann nun bis zum Start der Nachtbehandlung seinen Alltagsaktivitäten nachgehen. Wie im erfindungsgemäßen stand-by Modus zwischen Aufrüsten und erstem Auslauf 5 wartet in der Zeit zwischen dem ersten Einlauf 2 und dem Start der Nachtbehandlung das Dialysegerät dabei mit gefülltem Set auf die Fortsetzung. In dieser Zeit wird deshalb im Set ein Überdruck gegen Verkeimung gehalten. Darüber hinaus werden die Ventile regelmäßig bewegt, damit es aufgrund der Wartezeit zu keiner Sackbildung kommt. Durch den erfindungsgemäßen stand-by Modus zwischen Aufrüsten und erstem Auslauf 5 ist es bei diesem alternativen Verfahren dabei z.B. möglich, das Behandlungsgerät bereits am Morgen aufzurüsten und dann in den stand-by Modus zu versetzten. Die für den Behandlungszyklus am Tage benötigte Zeit wird so nicht noch unnötig durch das nach dem Stand der Technik notwendige Aufrüsten direkt vor Behandlungsbeginn verlängert. Damit ergibt sich für den Patienten eine enorme Flexibilisierung seines Tagesablaufs und damit eine stark erhöhte Lebensqualität.

## Patentansprüche

1. Verfahren zur Vorbereitung einer Behandlungsmaschine zur Behandlung einer medizinischen Flüssigkeit, welche eine Ankoppelfläche mit Aktoren (20) umfasst, wobei eine Kassette aus einem Hartteil mit flüssigkeitsführenden Kanälen (21), welche von einer flexiblen Folie (22) abgedeckt sind, an die Ankoppelfläche der Behandlungsmaschine ankoppelbar ist, so dass die Aktoren (20) durch Herabdrücken der flexiblen Folie (22) in die Kassette den Flüssigkeitsfluss in den flüssigkeitsführenden Kanälen (21) steuern können,
**dadurch gekennzeichnet,**
**dass** nach dem Aufrüsten der Behandlungsmaschine und vor dem Behandlungsbeginn in einen Stand-By Modus geschaltet wird bei welchem die Aktoren (20) abwechselnd bewegt werden, um die flexible Folie (22) zu entlasten.

2. Verfahren nach Anspruch 1, wobei der Stand-By Modus 10 Stunden, vorteilhafterweise 15 Stunden beibehalten wird.

3. Verfahren nach Anspruch 1, wobei im Stand-By Modus die Aktoren (20) so bewegt werden, dass keine vorher verschlossenen Flusswege geöffnet werden.

4. Verfahren nach Anspruch 1, wobei im Stand-By Modus die Aktoren (20) in regelmäßigen Abständen, vorteilhafterweise in Abständen von 5 bis 20 Minuten, bewegt werden.

5. Verfahren insbesondere nach Anspruch 1, wobei im Stand-By Modus ein Überdruck im System hergestellt wird.

6. Behandlungsmaschine zur Behandlung einer medizinischen Flüssigkeit, mit einer Ankoppelfläche mit Aktoren (20) und mit einer Steuerung, wobei eine Kassette aus einem Hartteii mit flüssigkeitsführenden Kanälen (21), welche von einer flexiblen Folie (22) abgedeckt sind, an die Ankoppelfläche der Behandlungsmaschine ankoppelbar ist, so dass die Aktoren (20) durch Herabdrücken der flexiblen Folie (22) in die Kassette den Flüssigkeitsfluss in den flüssigkeitsführenden Kanälen (21) steuern können,
**dadurch gekennzeichnet,**
**dass** die Steuerung einen nach dem Aufrüsten der Behandlungsmaschine und vor dem Behandlungsbeginn einschaltbaren Stand-By Modus aufweist, bei welchem die Aktoren (20) abwechselnd bewegt werden, um die flexible Folie (22) zu entlasten.

7. Behandlungsmaschine nach Anspruch 6, wobei im Stand-By Modus die Aktoren (20) so bewegt werden, dass keine vorher verschlossenen Flusswege geöffnet werden.

8. Behandlungsmaschine nach Anspruch 6, wobei im Stand-By Modus die Aktoren (20) in regelmäßigen Abständen, vorteilhafterweise in Abständen von 5 bis 20 Minuten bewegt werden.

9. Behandlungsmaschine insbesondere nach Anspruch 6, wobei die Steuerung im Stand-By Modus ein Überdruck im System herstellt.

10. Behandlungsmaschine nach Anspruch 6, wobei die Steuerung nach dem Aufrüsten automatisch in den Stand-By Modus schaltet, insbesondere wenn nicht innerhalb einer vorgegebenen Zeit mit der Behandlung begonnen wird.

11. Verfahren nach einem der Ansprüche 1-5 oder Behandlungsmaschine nach einem der Ansprüche 6-10, wobei die Behandlungsmaschine eine Behandlungsmaschine zur Peritonealdialyse ist, insbesondere eine Behandlungsmaschine zur automatischen Peritonealdialyse.

## Claims

1. A method for the preparation of a treatment machine for the treatment of a medical liquid comprising a coupling surface with actuators (20), with a cassette comprising a hard part with liquid conducting passages (21) which are covered by a flexible film (22) being able to be coupled to the coupling surface of the treatment machine so that the actuators (20) can control the liquid flow in the liquid conducting passages (21) by pressing the flexible film (22) down into the cassette,
**characterized in that**
a switch is made after the setting up of the treatment machine and before the start of treatment into a stand-by mode in which the actuators (20) are alternately moved to relieve the flexible film (22).

2. A method in accordance with claim 1, wherein the stand-by mode is maintained for 10 hours, advantageously for 15 hours.

3. A method in accordance with claim 1, wherein the actuators (20) are moved in the stand-by mode so that no previously closed flow paths are opened.

4. A method in accordance with claim 1, wherein the actuators (20) are moved at regular intervals, advantageously at intervals of 5 to 20 minutes, in the stand-by mode.

5. A method, in particular in accordance with claim 1, wherein an overpressure is established in the system in the stand-by mode.

6. A treatment machine for the treatment of a medical liquid comprising a coupling surface with actuators (20) and with a control, wherein a cassette comprising a hard part with liquid conducting passages (21) which are covered by a flexible film (22) can be coupled to the coupling surface of the treatment machine so that the actuators (20) can control the liquid flow into the liquid conducting passages (21) by pressing the flexible film (22) down into the cassette,
**characterized in that**
the control has a stand-by mode which can be switched on after the setting up of the treatment machine and before the start of treatment in which the actuators (20) are alternately moved to relieve the flexible film (22).

7. A treatment machine in accordance with claim 6, wherein the actuators (20) are moved in the stand-by mode such that no previously closed flow paths are opened.

8. A treatment machine in accordance with claim 6, wherein the actuators (20) are moved at regular intervals, advantageously at intervals of 5 to 20 minutes, in the stand-by mode.

9. A treatment machine, in particular in accordance with claim 6, wherein the control establishes an overpressure in the system in the standby mode.

10. A treatment machine in accordance with claim 6, wherein the control automatically switches into the stand-by mode after the setting up, in particular if the treatment is not started within a predetermined time.

11. A method in accordance with one of the claims 1 to 5 or a treatment machine in accordance with one of the claims 6 to 10, wherein the treatment machine is a treatment machine for peritoneal dialysis, in particular a treatment machine for automatic peritoneal dialysis.

## Revendications

1. Procédé pour préparer une machine de traitement pour le traitement d'un liquide médical, qui comporte une surface de rattachement avec des actionneurs (20), une cassette constituée d'une partie dure dotée de canaux (21) véhiculant du liquide, qui sont recouverts d'un film (22) flexible, pouvant être rattachée à la surface de rattachement de la machine de traitement, de sorte que les actionneurs (20) peuvent commander le flux de liquide dans les canaux (21) véhiculant du liquide par abaissement et enfoncement du film (22) flexible dans la cassette,
**caractérisé en ce que,**
avant l'équipement de la machine de traitement et avant le début du traitement, on commute dans un mode veille, dans lequel les actionneurs (20) sont déplacés alternativement pour décharger le film (22) flexible.

2. Procédé selon la revendication 1, le mode veille étant conservé pendant 10 heures, de préférence pendant 15 heures.

3. Procédé selon la revendication 1, les actionneurs (20) étant déplacés dans le mode veille de telle sorte qu'aucun chemin d'écoulement fermé auparavant ne soit ouvert.

4. Procédé selon la revendication 1, les actionneurs (20) étant déplacés dans le mode veille à intervalles réguliers, de préférence à des intervalles de 5 à 20 minutes.

5. Procédé en particulier selon la revendication 1, une surpression étant établie dans le système en mode veille.

6. Machine de traitement pour le traitement d'un liquide médical, dotée d'une surface de rattachement avec des actionneurs (20) et avec une commande, une cassette à base d'une partie dure dotée de canaux (21) véhiculant du liquide, qui sont recouverts d'un film (22) flexible pouvant être rattachée à la surface de rattachement de la machine de traitement, de sorte que les actionneurs (20) peuvent commander le flux de liquide dans les canaux (21) véhiculant du liquide par l'abaissement et l'enfoncement du film (22) flexible dans la cassette,
**caractérisé en ce que**
la commande présente un mode veille commutable avant l'équipement de la machine de traitement et le début du traitement, dans lequel les actionneurs (20) sont déplacés alternativement pour décharger le film (22) flexible.

7. Machine de traitement selon la revendication 6, les actionneurs (20) étant déplacés dans le mode veille de telle sorte qu'aucun chemin d'écoulement fermé auparavant ne soit ouvert.

8. Machine de traitement selon la revendication 6, les actionneurs (20) étant déplacés en mode veille à des intervalles réguliers, de préférence à des intervalles de 5 à 20 minutes.

9. Machine de traitement en particulier selon la revendication 6, la commande établissant une surpression dans le système en mode veille.

10. Machine de traitement selon la revendication 6, la commande commutant automatiquement dans le mode de veille après l'équipement, en particulier si l'on ne démarre pas le traitement en l'espace d'un temps prédéfini.

11. Procédé selon l'une quelconque des revendications 1 à 5 ou machine de traitement selon l'une quelconque des revendications 6 à 10, la machine de traitement étant une machine de traitement pour la dialyse péritonéale, en particulier une machine de traitement pour la dialyse péritonéale automatique.
